(19) Europäisches Patentamt
European Patent Office
Office européen des brevets

(11) Publication number: **0 170 214 B1**

# (12) EUROPEAN PATENT SPECIFICATION

(45) Date of publication of patent specification: **13.09.89**

(51) Int. Cl.$^4$: **C 07 D 213/46,** C 07 D 401/06

(21) Application number: **85109363.3**

(22) Date of filing: **25.07.85**

## (54) The preparation of 1,3-Dihydro-4-pyridoyl-2H-imidazol-2-ones.

(30) Priority: **30.07.84 US 635852**

(43) Date of publication of application:
**05.02.86 Bulletin 86/06**

(45) Publication of the grant of the patent:
**13.09.89 Bulletin 89/37**

(84) Designated Contracting States:
**AT BE CH DE FR GB IT LI LU NL SE**

(56) References cited:
**EP-A-0 059 948**

**CHEMICAL ABSTRACTS, vol. 84, no. 11, March 15, 1976, Columbus, Ohio, USA, E. POHJALA, "Indolizin derivatives", p. 431, abstract no. 74 071e**

**The file contains technical information submitted after the application was filed and not included in this specification**

(73) Proprietor: **MERRELL DOW PHARMACEUTICALS INC.**
**2110 East Galbraith Road**
**Cincinnati Ohio 45215-6300 (US)**

(72) Inventor: **Schnettler, Richard A.**
**9874 Forest Glen Drive**
**Cincinnati Ohio 45242 (US)**
Inventor: **King, Chi-Hsin R.**
**212 Nakoma Drive**
**Midland Michigan 48640 (US)**

(74) Representative: **Sgarbi, Renato et al**
**GRUPPO LEPETIT S.p.A. Patent and Trademark Department 34, Via Roberto Lepetit**
**I-21040 Gerenzano (Varese) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 170 214 B1

## Description

The present invention is directed to a process for producing 1,3-dihydro-4-pyridoyl-2*H*-imidazol-2-ones of the general Formula I

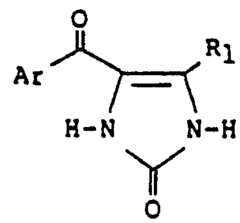

I

wherein $R_1$ is a hydrogen or a 1 to 4 carbon atom alkyl group and Ar is a 2-, 3- or 4-pyridyl group as well as the pharmaceutically acceptable salts thereof. These compounds and in particular 4-ethyl-1,3-dihydro-5-(4-pyridoyl)-2*H*-imidazol-2-one possess potent cardiotonic utility and are useful therapeutic agents in the treatment of cardiac failure.

These compounds have been prepared in the prior art by several methods. In one method an imidazol-2-one is reacted with a pyridoyl chloride or bromide or pyridine carboxylic acid or carboxylic acid anhydride in the presence of a Lewis acid catalyst, typically aluminum chloride. This process suffers from several serious drawbacks upon scale-up including extreme difficulty in mixing the solid aluminum complexes and resultant poor yields primarily due to difficulty in separating product from the solid mass of the reaction pot.

In another prior art reaction (see EP—A—59948) illustrated in Scheme I, a diketo-oxime of structure II is reduced to form an aminodiketone of structure III which upon reaction with a cyanate salt yields the structure I compounds.

SCHEME I

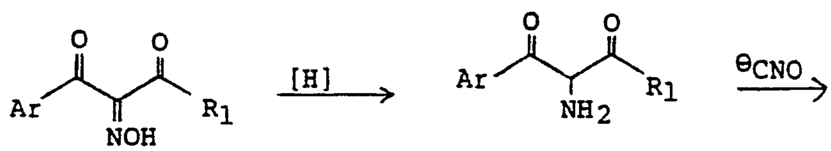


I

II III

Several difficulties are encountered when this process is employed. In particular, because the keto function adjacent to the pyridine ring in the structure II compounds is activated towards hydrogenation, this keto group is reduced along with the oxime group to yield the hydroxyaminoketones of formula IV.

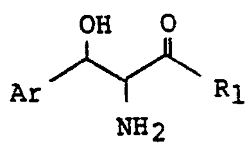

IV

This side reaction necessitates removal of the structure IV compounds from the hydrogenated reaction mixture and results in lowered overall yields of the desired structure I compounds.

Applicants have discovered that improved yields of the desired product of structure I can be obtained following the reaction path of scheme III.

**Scheme III**

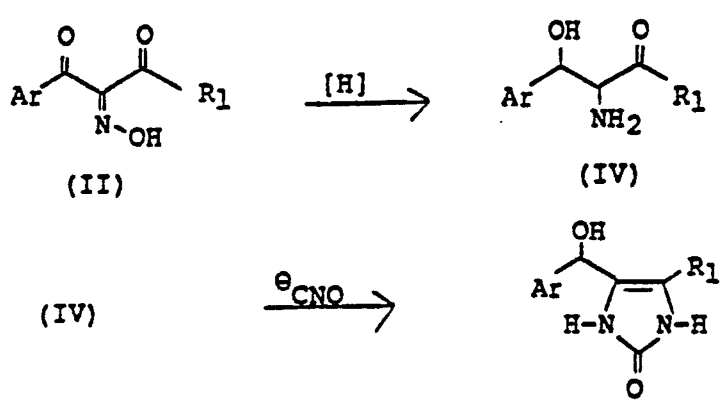


(II) (IV)

(IV)

(V)

(V) 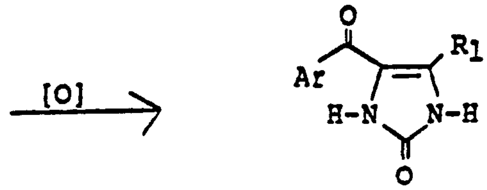


(I)

This improved reaction scheme eliminates the need to remove any of the hydroxyaminoketone of formula IV which forms by undesired side reaction in the scheme I process. Moreover, the reaction of the hydroxyaminoketone of formula IV with cyanate ion in scheme III proceeds with substantially greater yields of cyclized product than does the corresponding reaction of the diketoamino compound of structure III with cyanate ion in the scheme I process. Applicants have found, surprisingly, that the overall conversion of structure II compound to the desired pyridoylimidazol-2-one of structure I proceeds with substantially greater overall yields when the process of scheme III is utilized rather than that of scheme I. This occurs even though the scheme III process requires an additional step which has no counterpart in the closely related prior art process of scheme I, that is, scheme III requires the oxidation of the structure V alcohol to produce the desired pyridoylimidazol-2-one.

In accordance with this invention, pyridoylimidazol-2-ones of formula I are prepared by a three step process form the diketo-oximes of formula II as illustrated in scheme III. More particularly, the process of the present invention comprises reducing a diketo-oxime of structure II employing any suitable reducing agent to produce the hydroxyaminoketones of formula IV which after cyclization with a cyanate salt are oxidized to produce the desired pyridoylimidazol-2-one of formula I.

The applicants have discovered a process whereby a pyridoylimidazol-2-one of structure I can be prepared by sequentially reducing, cyclizing and oxidizing a diketo-oxime of structure II. The process of this invention is readily adapted to large scale batch production of structure I compounds in yields greater than other known methods.

As used herein, the term "a 1 to 4 carbon atom alkyl group" means a methyl, ethyl, propyl, isopropyl, n-butyl, or isobutyl group.

The starting materials, the diketo oximes of structure II, are readily prepared by any suitable procedure known in the art such as nitrosation of the corresponding diketone of formula VI wherein $R_1$ is a hydrogen or a 1 to 4 carbon atom alkyl group and Ar is a 2-, 3- or 4-pyridyl group.

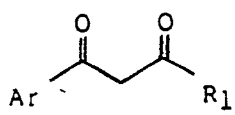

(VI)

Suitable nitrosation reactions are reviewed by O. Tousler in "Organic Reactions", Volume VII, pp. 327—377.

The reduction of the diketo-oximes of structure II to yield the hydroxyaminoketones of structure IV can be accomplished in any manner known to those skilled in the art using any suitable reducing agents. Applicants have employed as suitable reducing agents either a) hydrogen gas in the presence of a 10% Palladium on Charcoal catalyst using acetic acid solvent followed by a dilute acid workup or b) zinc metal and formic acid or acetic acid. It should be readily apparent, however, that there are innumerable other suitable reducing agents which will effect the desired conversion of the structure II diketo-oximes to the hydroxyaminoketones of structure IV. Because the hydroxyaminoketones are unstable when isolated as the free bases, it is advisable to isolate these structure IV compounds as acid addition salts. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Either the mono or the di-acid salts can be formed, and such salts can exist in either a hydrated or a substantially anhydrous form. In general, the acid addition salts of these compounds are crystalline materials which are soluble in water and various hydrophilic organic solvents and which in comparison to their free base forms, are substantially more stable.

Suitable means of reducing the structure II diketo-oximes include metal hydride reductions such as by using lithium aluminum hydride or sodium borohydride; catalytic reductions employing hydrogen gas and a metallic catalyst such as Raney nickel, platinum, palladium, rhodium, ruthenium and platinum oxide; and dissolving metal reductions employing lithium, sodium, potassium, calcium, zinc, magnesium, tin or iron in liquid ammonia or a low-molecular weight aliphatic amine or sodium, aluminum or zinc amalgam, zinc, tin or iron in a hydroxylic solvent or in the presence of an aqueous mineral or organic acid such as formic, acetic or hydrochloric acid.

Applicants have prepared the hydroxyaminoketones of structure IV by reduction of the structure II diketo-oximes with zinc dust in formic acid. The diketo-oxime to be reduced is dissolved in a suitable nonreactive solvent such as ethanol, isopropanol, n-butyl alcohol, isoamyl alcohol, water, an aqueous mineral acid such as hydrochloric acid or sulfuric acid or an organic acid, such as acetic acid, methanesulfonic acid or preferably formic acid. An acid such as hydrochloric acid or methanesulfonic acid may then be added, preferably by adding methanesulfonic acid to the dissolved reactant. The above solution is then slowly added to a slurry of the metal reductant in formic acid, preferably zinc dust, and the mixture stirred until the reaction is complete, typically from 5 minutes to 10 hours, preferably from about 1 to 2 hours. The reaction time will vary depending on the reactants, the solvent and the temperature which can be from 0° to 150°C preferably from 25° to 80°C. The product can be isolated from the reaction mixture either as the free base or preferably as an acid addition salt in any manner commonly employed by those skilled in the art. For example, if 10% methanol in isopropanol is added to the concentrated residue, the hydroxyaminoketone will precipitate from the solution and can then be separated by filtration.

Alternatively, applicants have reduced the diketo-oximes of structure II to prepare the hydroxyaminoketones of structure IV by utilizing hydrogen gas and a a palladium on carbon catalyst, preferably a 10% palladium on carbon catalyst. The diketo-oxime to be reduced is dissolved in a suitable solvent, a small amount of catalyst is added, preferably less than 10 per cent by weight of the amount of compound to be reduced, and the reaction allowed to proceed until 3 equivalents of hydrogen gas are taken up. The amount of time required will depend upon the compound to be reduced, the pressure of hydrogen gas used which can be from 1 to 10 atmospheres, preferably 1 atmosphere, the solvent and the temperature which can be from 0° to 50°C, preferably about 25°C. Suitable solvents include any nonreactive solvent including ethyl acetate, ethanol, water, or preferably acetic acid. When the reaction is complete, hydrochloric acid or any other suitable mineral acid is added to the reaction mixture which is then filtered to remove the solid catalyst. The hydroxyaminoketone or an acid addition salt thereof can then be recovered by any suitable method readily known to those skilled in the art including simple solvent removal.

The cyclization of the hydroxyaminoketones of structure IV with cyanate ion to produce the hydroxymethylimidazol-2-ones of structure V can be carried out by the ordinary artisan in any suitable manner. Typically, the hydroxyaminoketone and 1 to 5 molar equivalents, preferably about 2 molar equivalent of a cyanate salt are allowed to react for about 5 minutes to about 24 hours depending on the reactants, the solvent and the temperature which can be from −78 to about 100°C preferably from about 0° to 50°C. Suitable solvents for this reaction are any nonreactive solvent such as water or a water miscible solvent, for example, an organic acid such as acetic acid; an alcohol such as methanol or ethanol; or an ether such as diethyl ether, tetrahydrofuran or p-dioxan. Preferably, any nonaqueous solvent is mixed with water. The preferred solvent is water. Any source of cyanate ion may be utilized in the cyclization reaction. Applicants have utilized potassium cyanate but any simple alkali or alkaline earth salt such as lithium, sodium or calcium cyanate as well as a transition group metal cyanate would be useful.

The product of this reaction or an acid addition salt thereof can be isolated by any art-known procedure such as by conversion to the corresponding sodium or potassium salt and reprecipitation with carbon dioxide or a mineral acid such as dilute hydrochloric acid.

The final step of the scheme III process wherein a hydroxyimidazol-2-one of formula V is oxidized to yield the desired pyridoylimidazol-2-one of formula I can be carried out by any convenient manner by procedures readily known to those skilled in the art. Suitable oxidizing agents for use in the present process include manganese dioxide, aqueous acidic chromic acid in acetic acid or acetone; sodium dichromate in acetic acid; chromium trioxide pyridine complexes such as the Sarrett or Collins reagents; potassium permanganate with aqueous sulfuric and acetic acids; 40% peracetic acid; m-chloroperbenzoic acid; tetrachlorobenzoquinone (chloranil); 2,3-dichloro-5,6-dicyano-1,4-benzoquinone (DDQ); and N-haloimides preferably N-chlorosuccinimide.

Applicants prefer to oxidize the hydroxymethylimidazol-2-ones of structure V by reaction with an N-haloimide such as 1,3-dibromo-5,5-dimethylhydantoin, 1,3-dichloro-5,5-dimethylhydantoin, N-chloroacetamide, N-bromosuccinimide or preferably N-chlorosuccinimide. These oxidations are performed by dissolving the compound to be oxidized in a suitable solvent to which 1 to 5 molar equivalents, preferably about 1 molar equivalent, of the N-haloimide is added. The reaction temperature can be from about −78°C to about 80°C and will require from 1/2 hour to about 48 hours to be complete depending on the reactants, the solvent and other reaction conditions. Suitable solvents include any nonreactive solvents such as dimethylacetamide, methanol, dimethylformamide or preferably dimethylformamide-methanol cosolvent. The resulting product of Structure I can be isolated in any appropriate manner generally known to those skilled in the art such as by precipitation and subsequent recrystallization.

Applicants have also oxidized the hydroxymethylimidazolones of structure V by reaction with manganese dioxide. The compound to be oxidized is dissolved in any suitable solvent to which one or more molar equivalents, preferably 2 or 3 equivalents, of manganese dioxide are added and is allowed to react for 15 minutes to 10 hours, preferably about 1 or 2 hours, depending on the reactant, the solvent, and the temperature which can be from 0° to 150°, preferably about 25° to 80°C. Suitable solvents include pentane, chloroform, methylene chloride, benzene, acetone or preferably acetic acid. The pyridoylimidazol-2-one can be isolated from the reaction mixture by any procedure commonly employed by those skilled in the art. For example, applicants have isolated the product by filtration and solvent removal.

The following specific Examples more clearly illustrate the process of making and using this invention and set forth the best mode contemplated by the inventors for carrying out their invention.

Example 1

Preparation of 1-(4-pyridyl)-1-Hydroxy-2-Amino-3-Ketopetane

In 1000 ml acetic acid are dissolved 23.0 g (0.11 mol) of 1-(4-pyridyl)-1,3-diketo-2-oximinopentane. The solution is charged with 1.0 g of 10% palladium on carbon and hydrogenated until three equivalents of hydrogen were taken up. The mixture was acidified with 18.5 ml of 12N hydrochloric acid, filtered and the solvent evaporated to give the title compound as the dihydrochloric acid salt; mp. 225°C.

Following the procedure of Example 1 above but substituting:
1-(2-pyridyl)-1,3-diketo-2-oximinopentane;
1-(4-pyridyl)-1,3-diketo-2-oximinobutane;
1-(3-pyridyl)-1,3-diekto-2-oximinopropane;
1-(4-pyridyl)-1,3-diketo-4-methyl-2-oximinopentane; or
1-(2-pyridyl)-1,3-diketo-2-oximinoheptane;
for the
1-(4-pyridyl-1,3-diketo-2-oximinopentane
results in:
1-(2-pyridyl)-1-hydroxy-2-amino-3-ketopentane;
1-(4-pyridyl)-1-hydroxy-2-amino-3-ketobutane;
1-(3-pyridyl)-1-hydroxy-2-amino-3-ketopropane;
1-(4-pyridyl)-1-hydroxy-2-amino-4-methyl-3-ketopentane; or
1-(2-pyridyl)-1-hydroxy-2-amino-3-ketoheptane;
respectively.

Example 2

Preparation of 1-(4-pyridyl)-1-hydroxy-2-amino-3-ketopentane

In 20 ml acetic acid is dissolved 1.0 g of 1-(4-pyridyl)-1,3-diketo-2-oximinopentane with heat (50°C). The solution is acidified with dry hydrogen chloride and 1.0 g of zinc dust is slowly added. The mixture is stirred for one hour and cooled. Dry ether is added to the mixture and the title compound precipitates from the solution as a crude solid. This may be used in subsequent steps without purification.

Following the above procedure but employing:
1-(3-pyridyl)-1,3-diketo-4-methyl-2-oximinohexane;
1-(4-pyridyl)-1,3-diketo-2-oximinoheptane;
1-(3-pyridyl)-1,3-diketo-4-methyl-2-oximinopentane;
in place of
1-(4-pyridyl)-1,3-diketo-2-oximinopentane;
results in
1-(3-pyridyl)-1-hydroxy-2-amino-4-methyl-3-ketohexane;
1-(4-pyridyl)-1-hydroxy-2-amino-3-ketoheptane; or
1-(3-pyridyl)-1-hydroxy-2-amino-4-methyl-3-ketopentane;
respectively.

Example 3

Preparation of 1-(4-Pyridyl)-1-hydroxyl-2-amino-3-ketopentane

In 37.8 kg of 88% formic acid were dissolved 7.5 kg (91% pure, 36.37 mole); of 1-(4-pyridyl)-1,3-diketo-2-oximinopentane and 7.0 kg of methanesulfonic acid. The resulting solution was slowly added to a slurry of 8.3 kg of zinc powder in 35.7 of formic acid. The reaction temperature was kept at about 60°C by proper cooling and slow addition. The mixture was allowed to stir at 55°C for 2 hours and then cooled to 20°C. The solid zinc formate was filtered off. To the formic acid filtrate was added 3.6 kg of methanesulfonic acid. The formic acid was removed at the reduced pressure 5.3 kPa (40 mm Hg) and 70°C. To the residue was added a solution of 5.9 kg of methanol and 53.3 kg of isopropanol and stirred at 20°C for 4 hours. Solid material was collected by centrifugation, washed with 12.5 kg of 10% methanol in isopropanol giving 11.0 kg, 87% yield, of the title compound as dimethanesulfonate salt after drying.

Example 4

Preparation of 4-Ethyl-1,3-dihydro-5-[hydroxy(4-pyridyl)-methyl]-2H-imidazol-2-one

In 100 ml water is dissolved 29.0 g (0.11 mol) of 1-(4-pyridyl)-1-hydroxy-2-amino-3-ketopentane dihydrochloride and 17.9 g (0.22 mol) of potassium cyanate. The solution is warmed to 50°C for 10 minutes and then allowed to stand at room temperature for 10 hours, cooled and the solid collected to give the title compound; m.p. 234—36°C.

Following the procedure described above in Example 4 but using:
1-(2-pyridyl)-1-hydroxy-2-amino-3-ketopentane;
1-(4-pyridyl)-1-hydroxy-2-amino-3-ketobutane;
1-(3-pyridyl)-1-hydroxy-2-amino-3-ketopropane;
1-(4-pyridyl)-1-hydroxy-2-amino-4-methyl-3-ketopentane;
1-(2-pyridyl)-1-hydroxy-2-amino-3-ketoheptane;
1-(3-pyridyl)-1-hydroxy-2-amino-4-methyl-3-ketohexane;
1-(4-pyridyl)-1-hydroxy-2-amino-3-ketoheptane; or
1-(3-pyridyl)-1-hydroxy-2-amino-4-methyl-3-ketopentane;
instead of
1-(4-pyridyl)-1-hydroxy-2-amino-3-ketopentane
results in:
1,3-dihydro-4-ethyl-5-[hydroxy(2-pyridyl)methyl]-2*H*-imidazol-2-one;
1,3-dihydro-4-[hydroxy(4-pyridyl)methyl]-5-methyl-2*H*-imidazol-2-one;
1,3-dihydro-4-[hydroxy(3-pyridyl)methyl]-2*H*-imidazol-2-one;
1,3-dihydro-4-[hydroxy(4-pyridyl)methyl]-5-(1-methyl)ethyl-2*H*-imidazol-2-one;
4-butyl-1,3-dihydro-5-[hydroxy(2-pyridyl)methyl]-2*H*-imidazol-2-one;
1,3-dihydro-4-[hydroxy(3-pyridyl)methyl]-5-(1-methyl)-propyl-2*H*-imidazol-2-one;
4-butyl-1,3-dihydro-5-[hydroxy(4-pyridyl)methyl]-2*H*-imidazol-2-one; or
1,3-dihydro-4-[hydroxy-(3-pyridyl)methyl]-5-(1-methyl)-ethyl-2*H*-imidazol-2-one;
respectively.

## Example 5

1,3-Dihydro-4-Ethyl-5-(4-pyridoyl)-2H-imidazol-2-one

In 25 ml acetic acid is dissolved 2.15 g (0.009 mol) of compound I and heated to 50°C. Slowly 0.55 g (0.006 mol) of manganese dioxide is added to the solution and heating and stirring are continued for 30 minutes. The solution is filtered and the solvent evaporated. The residue is dissolved in dilute (10%) hydrochloric acid and the pH is adjusted to pH 4 with sodium bicarbonate. A solid separates which is the title compound. The material may be purified by recrystallization from ethanol; m.p. 264°C.

Employing the procedure of Example 4 above but substituting:
1,3-dihydro-4-ethyl-5-[hydroxy(2-pyridyl)methyl]-2*H*-imidazol-2-one;
1,3-dihydro-4-[hydroxy(4-pyridyl)methyl]-5-methyl-2*H*-imidazol-2-one;
1,3-dihydro-4-[hydroxy(3-pyridyl)methyl]-2*H*-imidazol-2-one;
1,3-dihydro-4-[hydroxy(4-pyridyl)methyl]-5-(1-methyl)ethyl-2*H*-imidazol-2-one;
4-butyl-1,3-dihydro-5-[hydroxy(2-pyridyl)methyl]-2*H*-imidazol-2-one;
1,3-dihydro-4-[hydroxy(3-pyridyl)methyl]-5-(1-methyl)-propyl-2*H*-imidazol-2-one;
4-butyl-1,3-dihydro-5-[hydroxy(4-pyridyl)methyl]-2*H*-imidazol-2-one; or
1,3-dihydro-4-[hydroxy(3-pyridyl)methyl]-5-(methyl)-ethyl-2*H*-imidazol-2-one,
for
1,3-dihydro-4-ethyl-5-[hydroxy(4-pyridyl)methyl]-2*H*-imidazol-2-one,
results in:
1,3-dihydro-4-ethyl-5-(2-pyridoyl)-2*H*-imidazol-2-one;
1,3-dihydro-4-methyl-5-(4-pyridoyl)-2*H*-imidazol-2-one;
1,3-dihydro-4-(3-pyridoyl)-2*H*-imidazol-2-one;
1,3-dihydro-4-(1-methyl)ethyl-5-(4-pyridoyl)-2*H*-imidazol-2-one;
4-butyl-1,3-dihydro-5-(2-pyridoyl)-2*H*-imidazol-2-one;
1,3-dihydro-4-(1-methyl)propyl-5-(3-pyridoyl)-2*H*-imidazol-2-one;
4-butyl-1,3-dihydro-5-(4-pyridoyl-2*H*-imidazol-2-one;
1,3-dihydro-4-(1-methyl)ethyl-5-(3-pyridoyl)-2*H*-imidazol-2-one;
respectively.

## Example 6

1,3-Dihydro-4-Ethyl-5-(4-pyridoyl)-2H-imidazol-2-one

To a slurry of 4.6 kg of 4-ethyl-1,3-dihydro-5-[hydroxy(4-pyridyl)methyl]-2*H*-imidazol-2-one in 2.9 kg of methanol and 14.0 kg of dimethylformamide was added a solution of 2.8 kg of N-chlorosuccinimide in 17.0 kg of dimethylformamide over a 2-hour period at 0°C. The resulting mixture was stirred at 0°C for 5 hours and then warmed up to 60°C for 3 hours. To the reaction mixture was added a solution of 1.7 kg of sodium acetate and 0.39 kg of sodium metabisulfite in 5.5 kg of water and stirred at 25°C for 4 hours. The resulting mixture was concentrated by vacuum distillation (at 80°C and 32kPa (24 mm Hg)) to remove 21 kg of solvents. To the concentrated solution was added 17.5 kg of water with stirring and cooled at −4°C for 12 hours. Solid material was collected by centrifuge to give 3.3 kg (72% yield, 99% purity) of the title compound after drying.

**Claims**

1. A process for the preparation of a pyridoylimidazol-2-one of the formula I

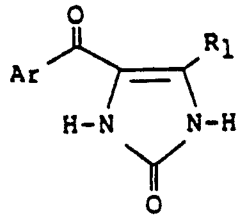

I

wherein Ar is a 2-, 3- or 4-pyridyl group and $R_1$ is hydrogen or a 1 to 4 carbon atom alkyl group which comprises:

a) reducing a diketo-oxime of the formula

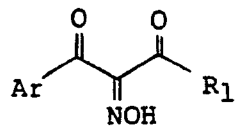

wherein Ar and $R_1$ are as defined above to produce an hydroxyiminoketone of the formula

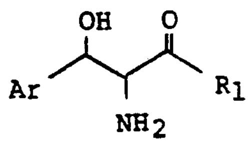

wherein Ar and $R_1$ are as defined above;

b) cyclizing the thus produced hydroxyiminoketone by reaction with a cyanate ion to produce an hydroxymethylimidazol-2-one of the formula

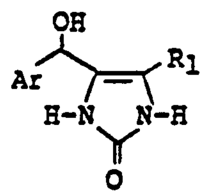

wherein Ar and $R_1$ are as defined above; and

c) oxidizing the thus produced hydroxymethylimidazol-2-one to produce the desired pyridoylimidazol-2-one and isolating the product.

2. A process of claim 1 wherein the hydroxymethylimidazol-2-one is oxidized with N-chlorosuccinimide.

3. A process of claim 1 wherein the hydroxymethylimidazol-2-one is oxidized with manganese dioxide.

4. A process of claim 1 wherein the diketo-oxime is reduced in step a) with hydrogen gas and a metal catalyst.

5. A process of claim 1 wherein the diketo-oxime is reduced in step a) with hydrogen gas and a palladium on carbon catalyst.

6. A process of claim 1 wherein the diketo-oxime is reduced by a dissolving metal.

7. A process of claim 1 wherein the diketo-oxime is reduced by a mixture consisting of zinc metal, formic acid and methanesulfonic acid.

8. A process of claim 1 wherein the cyanate in step b) is potassium cyanate.

9. A process of claim 1 wherein the diketo-oxime is reduced in step a) by a mixture consisting of zinc metal, formic acid and methanesulfonic acid and wherein the hydroxymethylimidazol-2-one is oxidized with N-chlorosuccinimide.

10. A process according to anyone of the above claims for preparing a compound of formula I wherein Ar is a 4-pyridyl group and $R_1$ is a hydrogen or a 1 to 4 carbon atom alkyl group.

11. A process according to anyone of the above claims for preparing a compound wherein $R_1$ is an ethyl group.

**Patentansprüche**

1. Verfahren zur Herstellung eines Pyridoylimidazol-2-ons der Formel I,

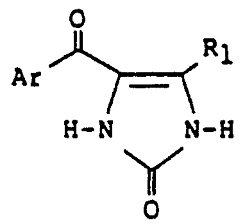

I

in der Ar eine 2-, 3- oder 4-Pyridylgruppe darstellt und $R_1$ ein Wasserstoffatom oder einen Alkylrest mit 1 bis 4 Kohlenstoffatomen bedeutet, welches umfaßt:

a) Reduktion eines Diketooxims der Formel,

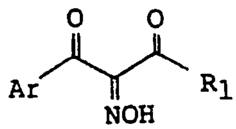

in der Ar und $R_1$ wie vorstehend definiert sind, wodurch ein hydroxyiminoketon der Formel,

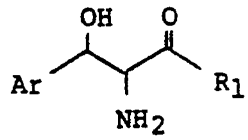

in der Ar und $R_1$ wie vorstehend definiert sind, erhalten wird;

b) Cyclisierung des so hergestellten Hydroxyiminoketons durch Umsetzung mit einem Cyanation, wordurch ein Hydroxymethylimidazol-2-on der Formel,

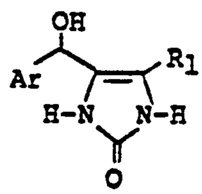

in der Ar und $R_1$ wie vorstehend definiert sind, erhalten wird; und

c) Oxidieren des so hergestellten Hydroxymethylimidazol-2-ons zur Herstellung des gewünschten Pyridoylimidazol-2-ons und Isolierung des Produktes.

2. Verfahren nach Anspruch 1, wobei das Hydroxymethylimidazol-2-on mit N-Chlorsuccinimid oxidiert wird.

3. Verfahren nach Anspruch 1, wobei das Hydroxymethylimidazol-2-on mit Mangandioxid oxidieirt wird.

4. Verfahren nach Anspruch 1, wobei das Diketooxim in Stufe a) mit Wasserstoffgas und einem Metall-Katalysator reduziert wird.

5. Verfahren nach Anspruch 1, wobei das Diketooxim in Stufe a) mit Wasserstoffgas und einem Palladium-auf-Kohle-Katalysator reduziert wird.

6. Verfahren nach Anspruch 1, wobei das Diketooxim durch ein auflösendes Metall reduziert wird.

7. Verfahren nach Anspruch 1, wobei das Diketooxim durch ein Gemisch, bestehend aus Zinkmetall, Ameisensäure und Methansulfonsäure, reduziert wird.

8. Verfahren nach Anspruch 1, wobei das Cyanat in Stufe b) Kaliumcyanat ist.

9. Verfahren nach Anspruch 1, wobei das Diketooxim in Stufe a) durch ein Gemisch, bestehend aus Zinkmetall, Ameisensäure und Methansulfonsäure, reduziert wird und wobei das Hydroxymethylimidazol-2-on mit N-Chlorsuccinimid oxidiert wird.

10. Verfahren nach einem der vorstehenden Ansprüche zur Herstellung einer Verbindung der Formel I, in der Ar eine 4-Pyridylgruppe bedeutet und $R_1$ ein Wasserstoffatom oder einen Alkylrest mit 1 bis 4 Kohlenstoffatomen darstellt.

11. Verfahren nach einem der vorstehenden Ansprüche zur Herstellung einer Verbindung, in der $R_1$ eine Äthylgruppe bedeutet.

**Revendications**

1. Un procédé de préparation d'une pyridoylimidazol-2-one de formule I

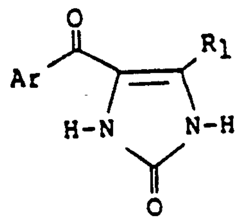 I

dans laquelle Ar est un groupe 2-, 3- ou 4-pyridyle et $R_1$ est un hydrogène ou un groupe alkyle de 1 à 4 atomes de carbone, ledit procédé comprenant:

a) la réduction d'une dicéto-oxime de formule

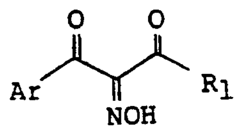

dans laquelle Ar et $R_1$ sont comme définis précédemment pour produire une hydroxyiminocétone de formule

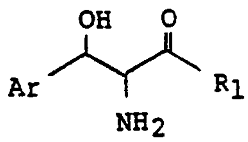

dans laquelle Ar et $R_1$ sont comme définis ci-dessus;

b) la cyclisation de l'hydroxyiminocétone ainsi produite par réaction avec un ion cyanate pour produire une hydroxyméthylimidazol-2-one de formule

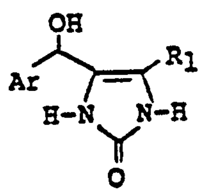

dans laquelle Ar et $R_1$ sont comme définis précédemment; et

c) l'oxydation de l'hydroxyméthylimidazol-2-one ainsi produite pour produire la pyridoylimidazol-2-one désirée et l'isolement du produit.

2. Un procédé selon la revendication 1 selon lequel l'hydroxyméthylimidazol-2-one est oxydée par le N-chlorosuccinimide.

3. Un procédé selon la revendication 1 selon lequel l'hydroxyméthylimidazol-2-one est oxydée par le dioxyde de manganèse.

4. Un procédé selon la revendication 1 selon lequel la dicéto-oxime est réduite dans l'étape a) par de l'hydrogène gazeux et un catalyseur métallique.

5. Un procédé selon la revendication 1 selon lequel la dicéto-oxime est réduite dans l'étape a) par de l'hydrogène gazeux et un catalyseur au palladium sur support de charbon.

6. Un procédé selon la revendication 1 selon lequel la dicéto-oxime est réduite par un métal dissolvant.

7. Un procédé selon la revendication 1 selon lequel la dicéto-oxime est réduite par un mélange constitué de zinc métallique, d'acide formique et d'acide méthanesulfonique.

8. Un procédé selon la revendication 1 selon lequel le cyanate de l'étape b) est du cyanate de potassium.

9. Un procédé selon la revendication 1 selon lequel la dicéto-oxime est réduite dans l'étape a) par un mélange constitué de zinc métallique, d'acide formique et d'acide méthanesulfonique et selon lequel l'hydroxyméthylimidazol-2-one est oxydée par le N-chlorosuccinimide.

10. Un procédé selon l'une quelconque des revendications précédentes pour préparer un composé de formule I dans laquelle Ar est un groupe 4-pyridyle et $R_1$ est l'hydrogène ou 1 groupe alkyle de 1 à 4 atomes de carbone.

11. Un procédé selon l'une quelconque des revendications précédentes pour préparer un composé dans lequel $R_1$ est un groupe éthyle.